# EUROPÄISCHE PATENTSCHRIFT

(11) **EP 1 948 737 B1**
(45) Veröffentlichungstag und Bekanntmachung des Hinweises auf die Patenterteilung: **24.07.2019**
(21) Anmeldenummer: 06828950.3
(22) Anmeldetag: 07.11.2006
(51) Int. Cl.: C09C 1/00, A61K 8/25, A61Q 1/02, A61Q 1/06, A61Q 1/10, A61Q 3/02, A61K 8/11, A61K 8/19

(54) **EFFEKTPIGMENT UND DESSEN VERWENDUNG IN DER KOSMETIK UND IM LEBENSMITTEL- UND PHARMABEREICH**
EFFECT PIGMENT AND USE THEREOF IN COSMETICS AND IN THE FOOD AND PHARMACEUTICAL INDUSTRIES
PIGMENT A EFFET ET SON UTILISATION EN COSMETIQUE AINSI QUE DANS LES SECTEURS ALIMENTAIRE ET PHARMACEUTIQUE

(30) Priorität: 18.11.2005 DE 102005055576
(43) Veröffentlichungstag der Anmeldung: 30.07.2008
(73) Patentinhaber: Merck Patent GmbH, 64293 Darmstadt (DE)
(72) Erfinder: PFAFF, Gerhard, 64839 Münster (DE); DIETZ, Johann, D-63218 Dietzenbach (DE); FOERDERER, Cornelia, 64646 Heppenheim (DE); PETSITIS, Xenia, 65719 Hofheim am Taunus (DE); WARTHE, Doreen, 64347 Griesheim (DE); HOCHSTEIN, Veronika, 76646 Bruchsal (DE)
(86) Internationale Anmeldenummer: PCT/EP2006/010669
(87) Internationale Veröffentlichungsnummer: WO 2007/057111

(56) Entgegenhaltungen:
- EP-A- 1 281 732
- EP-A- 1 681 318
- EP-A1- 0 960 912
- EP-A2- 1 045 014
- DE-A1- 10 354 763
- DE-A1-102004 045 352
- P. HOFFMANN, W. DUSCHEK: "Neue Effektpigmente" BERICHTSBAND DFO 41, Bd. 50, 1999, Seiten 123-132, XP008082167

## Beschreibung

Die vorliegende Erfindung betrifft lichtstabile Effektpigmente basierend auf dünnen transparenten SiO₂-Plättchen umhüllt mit einer Eisenoxidschicht und deren Verwendung in purer Form oder in Mischung mit anderen Farbmitteln und/oder Füllstoffen in kosmetischen Formulierungen und im Lebensmittel- und Pharmabereich.

Aus der WO 93/08237 sind Effektpigmente auf der Basis von transparenten Siliziumdioxid-Plättchen bekannt. Die dort beschriebenen Pigmente bestehen aus dünnen SiO₂-Plättchen (Dicke der Plättchen von 50 µm bis 5000 µm), die mit einer dünnen Metalloxidschicht oder mehreren Metalloxidschichten umhüllt sind. Anstelle von Metalloxiden können die SiO₂-Plättchen auch mit anderen Materialien wie Metallen, Sulfiden oder Nitriden umhüllt sein.

Aus der WO 2004/044060 A1, WO 2004/065492 A1 und EP 1556446 A1 sind beispielsweise mit Metalloxiden beschichtete SiO₂-Plättchen bekannt, wobei der Parameter z folgende Werte einnehmen kann: 0,7 ≤ z ≤ 2,0, 0,03 ≤ z ≤ 2,0 oder 0,7 ≤ z ≤ 1,8 bedeutet.

Die im Markt befindlichen metalloxidbeschichteten SiO₂-Plättchen mit Markennamen Xirona® der Fa. Merck KGaA bestehen aus SiO₂-Plättchen einheitlicher Dicke (± 5 nm) umhüllt mit einer Metalloxidschicht (TiO₂ oder Fe₂O₃) der Dicke von 10 nm bis 500 nm. Die Dicken der SiO₂-Plättchen liegen im Bereich von 200 nm bis 900 nm. Derartige SiO₂-Pigmente zeichnen sich je nach Dicke der eingesetzten Plättchen und der aufgebrachten Metalloxidschichten sowie nach Art des Metalloxids durch besonders intensive Interferenzfarben und/oder durch starke winkelabhängige Farbwechseleffekte aus. Letztere zeigen sich dabei so, dass ein Betrachter beim Wechsel seiner Beobachtungsposition zum pigmentierten Objekt unterschiedliche Farben wahrnimmt.

Farbstarke rote Effektpigmente auf Basis von SiO₂-Plättchen sind in der DE 102005002124 beschrieben. Diese Pigmente umfassen mit Eisenoxid beschichtete SiO₂-Plättchen, wobei die Gesamtdicke der Pigmente nicht größer als 500 nm (± 30 nm) ist.

DE 10 2004 045 352 A1 betrifft glänzende rote Interferenzpigmente basierend auf mehrfach beschichteten plättchenförmigen Substraten bzw. Substratgemischen.

DE 103 54 763 A1 offenbart Effektpigmente mit einem Aluminium- oder Aluminiumlegierungskern.

EP 1 281 732 A1 offenbart Mehrschichtpigmente mit alternierenden hoch- und niedrigbrechenden Schichten.

EP 1 045 014 A2 offenbart Pigmentmischungen auf der Basis von Mehrschichtpigmenten, die sich durch einen hohen Glanz aufzeichnen.

EP 0 960 912 A1 ist auf ein elektrisch leitfähiges Pigmentgemisch gerichtet.

Rote Effektpigmente auf der Basis von SiO₂-Plättchen zeichnen sich durch ihren hohen Glanz, ihre Farbreinheit und Farbstärke aus und sollten daher insbesondere für die Kosmetik und zur Einfärbung von Lebensmittel- und Pharmaprodukte interessant sein, sofern sie lichtstabil sind, nicht ausbluten/migrieren, weiches Hautgefühl vermitteln, ungiftig sind und hohes Deckvermögen besitzen.

Pigmente auf der Basis von SiO₂-Plättchen haben aber den Nachteil, dass sie aufgrund der hohen Transparenz nicht deckend sind. Aufgabe der vorliegenden Erfindung was es ein rotes Effektpigment auf der Basis von SiO₂-Plättchen bereit zu stellen, dass nicht nur lichtstabil ist, sondern auch ein vergleichsweise hohes Deckvermögen aufweist, sich sehr gut in das jeweilige Anwendungssystem einarbeiten lassen und gleichzeitig die optischen Eigenschaften, wie insbesondere der Glanz und die Farbreinheit, gar nicht oder nur unwesentlich beeinflusst werden. Sie sind chemisch sehr stabil sowohl in wässriger als auch in öliger Phase, d.h. sie bluten nicht aus. Daher sind sie sowohl für den Augen- als auch für den Lippenbereich einsetzbar.

Überraschenderweise wurden nun Effektpigmente gefunden, die neben einer außerordentlich hohen Farbstärke eine hohe Lichtstabilität aufweisen. Diese Pigmente zeichnen sich dadurch aus, dass sie auf transparenten undotierten SiO₂-Plättchen basieren, die mit einer Eisenoxidschicht der Dicke von 60-250 nm, vorzugsweise 60 - 150 nm, umhüllt sind, wobei alle SiO₂-Plättchen eine identische Dicke aufweisen und die Dicke der SiO₂-Plättchen 250-400 nm, vorzugsweise 270 bis 370 nm, beträgt. Derartige Pigmente zeigen im Suntest keine Farbänderungen und sind somit sehr lichtstabil.

Das Deckvermögen der erfindungsgemäßen Pigmente wird deutlich erhöht, sofern diese Pigmente in Kombination mit organischen und anorganischen Füllstoffen und/oder mit plättchenförmigen, nadelförmigen, sphärischen oder kristallinen Farbmitteln gemischt werden. Durch die Zumischung eines oder mehrerer Farbmittel zu diesen Effektpigmenten können Farbeffekte verstärkt und neuartige Farbeffekte erzielt werden. Weiterhin zeichnen sich die Pigmentgemische durch ihren hohen Glanz und ein sehr gutes Hautgefühl aus.

Gegenstand der Erfindung sind somit farbstarke und lichtstabile Effektpigmente gemäß Anspruch 1 basierend auf transparenten undotierten SiO₂-Plättchen, dadurch gekennzeichnet, dass sie mit einer Eisenoxidschicht der Dicke von 60-250 nm umhüllt sind und alle SiO₂-Plättchen eine identische Dicke aufweisen und die Dicke der SiO₂-Plättchen 250-400 nm beträgt.

Gegenstand der Erfindung ist weiterhin ein Pigmentgemisch bestehend aus mindestens zwei Komponenten A und B, wobei Komponente A die farbstarken und lichtstabilen Effektpigmente gemäß Anspruch 1 sind und Komponente B anorganische Pigmente, organische Pigmente, Farbstoffe und/oder Füllstoffe sind, die aus plättchenförmigen, nadelförmigen, sphärischen oder unregelmäßig geformten Partikeln bestehen.

Der herausragende koloristische Parameter ist die kräftige, reine rote Farbe der Pigmente verbunden mit einem optimalen Glanzeffekt. Das Eisenoxid liegt dabei als Fe₂O₃ vor. Die roten Effektpigmente besitzen Lab-Werte im Bereich von L = 20 bis 200; a = 20 bis 100; b = 10 bis 70 (ETA Messsystem, winkelabhängig) und sind daher hervorragend für die Kosmetik, insbesondere die dekorative Kosmetik, geeignet.

Gegenstand der Erfindung sind ebenfalls kosmetische Formulierungen, wie z. B. Make-ups, Presspuder, lose Puder, Lippenstifte, Lotions, Emulsionen, etc., die das erfindungsgemäße Pigmentgemisch enthalten. Weiterhin geeignet sind die Pigmentgemische für Einfärbungen und farbige Überzüge sowie zur Veredelung von Lebensmitteln und Pharmaprodukten, einschließlich OTC-Präparaten, wie z.B. Arzneimittelüberzüge von Tabletten, Dragees, Gelatinekapseln, etc.

Die roten Effektpigmente werden mit dem Farbmittel bzw. Füllstoff gemischt. Das Massenverhältnis von Komponente A zu Komponente B ist
99 : 1 bis 50 : 50, insbesondere
95 : 5 bis 70 : 30, ganz besonders bevorzugt 70 : 30 bis 50 : 50.

Die roten Effektpigmente besitzen folgenden Aufbau:
SiO₂-Plättchen der Dicke 250 - 400 nm, vorzugsweise 300 - 370 nm, insbesondere 340 - 365 nm +
Fe₂O₃-Schicht der Dicke 60 - 250 nm, vorzugsweise 60 - 150 nm, insbesondere 80 - 120 nm.

Die Dicken der SiO₂-Plättchen und der Eisenoxidschicht werden derart gewählt, dass die Gesamtdicke der roten Effektpigmente nicht größer als 500 ( ± 30) nm, vorzugsweise ≤ 440 ( ± 30) nm, insbesondere < 400 ( ± 30) nm ist.

Zur Erzielung spezieller Farbeffekte können in die Fe₂O₃-Schicht zusätzlich noch feinteilige Partikel im Nanometergrößenbereich eingebracht werden. Als geeignet dafür erweisen sich beispielsweise feinteiliges TiO₂ oder feinteiliger Kohlenstoff (Ruß) mit Teilchengrößen im Bereich von 10-250 nm. Durch die lichtstreuenden Eigenschaften derartiger Partikel kann gezielt auf Glanz und Deckvermögen Einfluss genommen werden.

Die Herstellung der SiO₂-Plättchen kann beispielsweise erfolgen, wie in der Offenlegungsschrift WO 93/08237 beschrieben.

Die Beschichtung der SiO₂-Plättchen mit Eisenoxidschichten kann nasschemisch und/oder durch CVD-Verfahren erfolgen. Vor dem Aufbringen der Eisenoxidschicht kann optional noch eine dünne dielektrische Schicht mit 1,4 < n < 2,7 abgeschieden werden. Eine solche Beschichtung auf SiO₂-Plättchen kann beispielsweise aus einer 2 - 20 nm dicken Al₂O₃-, ZnO- oder TiO₂-Schicht und darauf folgend einer koloristisch angepassten Fe₂O₃-Schicht bestehen.

Vorzugsweise wird das nasschemische Verfahren zur Herstellung der roten Effektpigmente verwendet, wobei die bekannten, zur Herstellung von Perlglanzpigmenten entwickelten, nasschemischen Beschichtungstechnologien angewendet werden können, die beispielsweise in den folgenden

Publikationen beschrieben sind: DE 14 67 468, DE 19 59 988, DE 20 09 566, DE 22 14 545, DE 22 15 191, DE 22 44 298, DE 23 13 331, DE 25 22 572, DE 31 37 808, DE 31 37 809, DE 31 51 343, DE 31 51 354, DE 31 51 355, DE 32 11 602, DE 32 35 017.

Zur Beschichtung werden die SiO₂-Plättchen in Wasser suspendiert und mit Eisenoxid durch Zugabe und Fällung entsprechender anorganischer Eisenverbindungen komplett beschichtet, d.h. umhüllt, wobei durch gleichzeitige Zugabe von Säure oder Base der für die Fällung von Eisenoxid, vorzugsweise Fe₂O₃, notwendige pH-Wert eingestellt und konstant gehalten wird und anschließend das beschichtete Substrat aus der wässrigen Suspension abgetrennt, getrocknet und gegebenenfalls kalziniert wird, und wobei die Schichtdicken der einzelnen Schichten so eingestellt werden, dass nach dem Trocknen und gegebenenfalls Kalzinieren die Dicke des Pigmentes nicht größer als 500 ( ± 30) nm, vorzugsweise < 440 ( ± 30) nm ist. Die Kalzinierungstemperatur liegt in der Regel zwischen 250 und 1000 °C, vorzugsweise zwischen 350 und 900 °C.

Prinzipiell sind für die Herstellung der erfindungsgemäßen Pigmente auch CVD-Verfahren zur Beschichtung von Partikeln mit Eisenoxid geeignet. Verfahren dieser Art sind zum Beispiel in W. Ostertag, Nachr. Chem. Tech. Lab 1994, 42, 849 beschrieben. Dabei ist es erforderlich, dass das Substrat während des Beschichtungsvorganges gleichmäßig in Bewegung gehalten wird, damit eine homogene Beschichtung aller Partikeloberflächen gewährleistet ist und das Substrat komplett umhüllt wird und keine offenen Kanten zurückbleiben.

Die roten Effektpigmente können auch zur Verbesserung der Licht-, Wetter- und chemischen Stabilität oder zur Erhöhung der Kompatibilität in unterschiedliche Medien noch mit einer organischen oder anorganischen Schutzschicht versehen sein. Als Nachbeschichtungen bzw. Nachbehandlungen kommen beispielsweise Silane, Silikone, adsorbierenden Silikone, Metallseifen, Aminosäuren, Lecithine, Fluorkomponenten, Polyethylene, Kollagen oder die in den DE 22 15 191, DE 31 51 354, DE 32 35 017 oder DE 33 34 598, EP 0 632 109, US 5,759,255, DE 43 17 019, DE 39 29 423, EP 0 492 223, EP 0 342 533, EP 0 268 918, EP 0 141 174, EP 0 764 191, WO 98/13426 oder EP 0 465 805 beschriebenen Verfahren in Frage. Durch diese Nachbeschichtung wird die chemische und photochemische Stabilität weiter erhöht oder die Handhabung des roten Pigments, insbesondere die Einarbeitung in unterschiedliche Medien, erleichtert. Zur Verbesserung der Benetzbarkeit, Dispergierbarkeit und/oder Verträglichkeit mit den Anwendermedien können beispielsweise funktionelle Beschichtungen aus Al₂O₃ oder ZrO₂ oder deren Gemische auf die Pigmentoberfläche aufgebracht werden. Weiterhin sind organische Nachbeschichtungen möglich, z.B. mit Silanen, wie beispielsweise beschrieben in der EP 0090259, EP 0 634 459, WO 99/57204, WO 96/32446, WO 99/57204, U.S. 5,759,255, U.S. 5,571,851, WO 01/92425 oder in J.J. Ponjeé, Philips Technical Review, Vol. 44, No. 3, 81 ff. und P.H. Harding J.C. Berg, J. Adhesion Sci. Technol. Vol. 11 No. 4, S. 471-493. Die zusätzlich aufgebrachten Stoffe machen nur etwa 0,1 bis 5 Gew.%, vorzugsweise 0,5 bis 3,0 Gew.%, des gesamten Pigments aus.

Die Nachbeschichtung der roten Effektpigmente kann direkt in einem Eintopfverfahren an die Fe₂O₃-Beschichtung der SiO₂-Plättchen erfolgen. Es ist aber auch möglich das rote Effektpigment zunächst zu isolieren, gegebenenfalls zu trocken und zu kalzinieren und anschließend die Nachbeschichtung aufzubringen.

Erfindungsgemäße Pigmentgemische enthalten neben dem roten Effektpigment (Komponente A) einen Füllstoff und/oder ein Farbmittel der Komponente B.

Als Komponente B für die erfindungsgemäße Pigmentmischung sind alle dem Fachmann bekannten plättchenförmigen, nadelförmigen, sphärischen und kristallinen Farbmittel oder Füllstoffe geeignet, insbesondere solche, die eine Partikelgröße von 0,001 bis 10 µm, vorzugsweise 0,01 bis 1 µm, aufweisen. Bevorzugt enthalten die erfindungsgemäßen Pigmentgemische als Farbmittel Absorptionspigmente und als Füllstoffe plättchenförmige oder sphärische Pulver. Komponente B sind vorzugsweise beschichtete oder unbeschichtete SiO₂-Kugeln. Mit ein oder mehreren Metalloxiden beschichtete SiO₂-Kugeln sind z.B. aus der EP 0 803 550 A2 bekannt.

Bevorzugte Pigmentgemische enthalten neben Komponente A als Farbmittel (Komponente B) insbesondere ein Perlglanzpigment, einschließlich Mehrschichtpigmente oder Interferenzpigmente. Als Perlglanzpigmente werden Pigmente auf der Basis plättchenförmiger, transparenter oder semitransparenter Substrate aus z.B. Schichtsilikaten, wie etwa natürlichem oder synthetischem Glimmer, Talkum, Sericit, Kaolin oder anderen silikatischen Materialien verwendet, die mit farbigen oder farblosen Metalloxiden wie z.B. TiO₂, Titansuboxide, Titanoxinitride, Fe₂O₃, Fe₃O₄, FeOOH, SnO₂, Cr₂O₃, ZnO, CuO, NiO und anderen Metalloxiden allein oder in Mischung in einer einheitlichen Schicht oder in aufeinanderfolgenden Schichten beschichtet sind. Besonders bevorzugte Perlglanzpigmente und Interferenzpigmente basieren auf einem synthetischem oder natürlichem Glimmerplättchen (synthetischer oder natürlicher Glimmer), Glas-, Al₂O₃-, SiO₂-Plättchen, ferner Fe₂O₃-Plättchen, das mit Titandioxid (Rutil oder Anatas), Fe₂O₃, einem Gemisch aus TiO₂ und Fe₂O₃ oder mit Fe₃O₄ beschichtet ist.

Perlglanzpigmente sind z.B. aus den deutschen Patenten und Patentanmeldungen 14 67 468, 19 59 998, 20 09 566, 22 14 454, 22 15 191, 22 44 298, 23 13 331, 25 22 572, 31 37 808, 31 37 809, 31 51 343, 31 51 354, 31 51 355, 32 11 602, 32 35 017 und P 38 42 330 bekannt und im Handel erhältlich, z.B. unter den Marken Iriodin®, Timiron®, Xirona® der Firma Merck KGaA, Darmstadt, Deutschland und/oder Rona, USA. Besonders bevorzugte Pigmentpräparationen enthalten TiO₂/Glimmer-, Fe₂O₃/Glimmer- und/oder TiO₂/Fe₂O₃-Glimmerpigmente. Die Perlglanzpigmente können zusätzlich noch eine Schicht aus Berliner Blau oder Carminrot auf der Oberfläche aufweisen.

Weiterhin bevorzugt sind beschichtete oder unbeschichtete BiOCI-Pigmente, mit TiO₂ und/oder Fe₂O₃ beschichtete SiO₂-, Glas- oder Al₂O₃-Plättchen. Die Beschichtung der SiO₂-Plättchen mit ein oder mehreren Metalloxiden kann z.B. erfolgen wie in der WO 93/08237 (naß-chemische Beschichtung) oder DE-OS 196 14 637 (CVD-Verfahren) beschrieben.

Die beispielsweise aus den deutschen Offenlegungsschriften DE 196 18 563, DE 196 18 566, DE 196 18 569, DE 197 07 805, DE 197 07 806, DE 197 46 067 bekannten Mehrschichtpigmente basieren auf einer plättchenförmigen, transparenten, farbigen oder farblosen Matrix, bestehend aus Glimmer (synthetisch oder natürlich), SiO₂-Plättchen, Glasplättchen, Al₂O₃-Plättchen, Polymerplättchen, und besitzen in der Regel eine Dicke zwischen 0,3 und 5 µm, insbesondere zwischen 0,4 und 2,0 µm. Die Ausdehnung in den beiden anderen Dimensionen beträgt üblicherweise zwischen 1 und 250 µm, vorzugsweise zwischen 2 und 100 µm, und insbesondere zwischen 5 und 40 µm. Die Mehrschichtpigmente bestehen aus der Matrix (Substrat) beschichtet mit Metalloxiden (mindestens 2). Die Beschichtung der Substratplättchen Glimmer, SiO₂-Plättchen, Glasplättchen, Al₂O₃-Plättchen mit mehreren Schichten erfolgt so, dass ein Schichtaufbau vorzugsweise bestehend aus alternierenden hoch- und niedrigbrechenden Schichten entsteht. Vorzugsweise enthalten die Mehrschichtpigmente 2, 3, 4, 5, 6 oder 7 Schichten, insbesondere 3, 4 oder 5 Schichten. Geeignete hochbrechende Metalloxide sind beispielsweise Titandioxid, Zirkonoxid, Zinkoxid, Eisenoxide, Eisen-Titan-Oxide (Eisentitanate) und/oder Chromoxid, insbesondere TiO₂ und/oder Fe₂O₃. Als niedrigbrechende Oxide kommen SiO₂ und Al₂O₃ zum Einsatz. Es kann hierfür jedoch auch MgF₂ oder ein organisches Polymer (z.B. Acrylat) eingesetzt werden. Die Beschichtung der Substratplättchen kann z.B. erfolgen wie in der WO 93/08237 (nasschemische Beschichtung) oder DE-OS-196 14 637 (CVD-Verfahren) beschrieben. Besonders bevorzugte Mehrschichtpigmente auf Basis von Glimmer (natürlich oder synthetisch), Glasplättchen, Al₂O₃-Plättchen, Fe₂O₃-Plättchen, SiO₂-Plättchen enthalten eine Schichtenfolge TiO₂ - SiO₂ - TiO₂. Das TiO₂ kann dabei in der Rutil- oder in der Anatasmodifikation vorliegen. Vorzugsweise liegt es als Rutil vor.

Bei den Interferenzpigmenten handelt es sich vorzugsweise um Pigmente auf der Basis von Glimmer, Glasplättchen, SiO₂-Plättchen, die mit farbigen oder farblosen Metalloxiden wie z.B. TiO₂, Titansuboxide, Titanoxinitride, Fe₂O₃, Fe₃O₄, SnO₂, Cr₂O₃, ZnO, CuO, NiO und anderen Metalloxiden allein oder in Mischung in einer einheitlichen Schicht oder in aufeinanderfolgenden Schichten beschichtet sind.

Sofern die genannten Perlglanz-, Interferenz- und Mehrschichtpigmente eine TiO₂-Schicht enthalten, kann diese in der Rutil- oder in der Anatasmodifikation vorliegen. Vorzugsweise liegt sie als Rutil vor. Die Rutilisierung ist dem Fachmann bekannt und kann beispielsweise erfolgen wie in der U.S. 4,038,099, U.S. 5,433,779, U.S. 6,626,989, WO 03/097749, U.S. 4,086,100, U.S. 4,867,794 beschrieben. Besonders bevorzugt ist die Rutilisierung unter Verwendung von Zinnoxid, wie z.B. in der U.S. 4,867,794 beschrieben.

Als plättchenförmige Farbmittel kommen vor allem Perlglanzpigmente insbesondere auf Basis von Glimmer, SiO₂-Plättchen oder Al₂O₃-Plättchen, die nur mit einer Metalloxidschicht umhüllt sind, Metalleffektpigmente (Al-Plättchen, Bronzen), optisch variable Pigmente (OVP's), Flüssigkristallpolymerpigmente (LCP's) oder holographische Pigmente in Frage.

Die Pigmente der Komponente B auf der Basis von SiO₂₋Plättchen sind selbstverständlich unterschiedlich zu dem Effektpigment der Komponente A.

Zu den sphärischen Farbmitteln zählen insbesondere TiO₂, eingefärbtes SiO₂, CaSO₄, Eisenoxide, Chromoxide, Ruß, organische Farbpigmente, wie z.B. Anthrachinon-Pigmente, Chinacridon-Pigmente, Diketopyrrolo-pyrrol-Pigmente, Phthalocyanin-Pigmente, Azopigmente, Isoindolin-Pigmente. Bei den nadelförmigen Pigmenten handelt es sich vorzugsweise um BiOCI, eingefärbte Glasfasern, α-FeOOH, organische Farbpigmente, wie z.B. Azopigmente, β-Phthalocyanin Cl Blue 15,3, Cromophtalgelb 8GN (Ciba-Geigy), Irgalith Blau PD56 (Ciba-Geigy), Azomethinkupferkomplex Cl Yellow 129, Irgazingelb 5GT (Ciba-Geigy).

Ebenso können nanoskalige Dielektrika zur Verbesserung des Hautgefühls beigemischt werden. Beispiele für derartige Beimischungen sind Al₂O₃, SiO₂, ZnO oder TiO₂, die üblicherweise in Mengen von 0,01 - 15 % der Formulierung zugegeben werden.

Die erfindungsgemäße Pigmentmischung ist einfach und leicht zu handhaben. Die Pigmentmischung kann durch einfaches Einrühren in das Anwendungssystem eingearbeitet werden. Die Komponenten A und B können dabei gleichzeitig, nacheinander oder als Gemisch dem Anwendungssystem zugegeben werden. Ein aufwendiges Mahlen und Dispergieren der Pigmente ist nicht erforderlich.

Die erfindungsgemäße Pigmentmischung kann zur Pigmentierung von Lebensmitteleinfärbungen, zur Veredlung von Lebensmitteln, z. B. Masse-Einfärbung oder als Überzug, in Arzneimittelüberzügen, z.B. bei Dragees und Tabletten, oder in kosmetischen Formulierungen, wie Lippenstifte, Lipgloss, Eyeliner, Lidschatten, Rouge, Sonnenschutz, Prä-Sun- und After-Sun-Präparate, Make-ups, Body Lotions, Badegele, Seifen, Badesalze, Zahnpasta, Haargele, (Volumen)Mascara, Nagellacke, Presspuder, Shampoos, lose Puder und Gele, etc., verwendet werden.

Die Konzentration der Pigmentmischung im zu pigmentierenden Anwendungssystem liegt in der Regel zwischen 0,01 und 70 Gew.%, vorzugsweise zwischen 0,1 und 50 Gew.% und insbesondere zwischen 1,0 und 10 Gew.%, bezogen auf den Gesamtfestkörpergehalt des Systems. Sie ist in der Regel abhängig vom konkreten Anwendungsfall und kann bei losen Pudern bis zu 100 % betragen. Die Einsatzkonzentration des erfindungsgemäßen Pigmentgemisches reicht von 0,01 Gew.% im Shampoo bis zu 70 Gew. % im Presspuder. Bei einer Mischung der roten Effektpigmente mit sphärischen Füllstoffen, z. B. SiO₂, kann die Konzentration bei 0,01-70 Gew.% in der Formulierung liegen. Die kosmetischen Produkte, wie z.B. Nagellacke, Lippenstifte, Presspuder, Shampoos, lose Puder und Gele, zeichnen sich durch besonders interessante Glanzeffekte aus.

Das erfindungsgemäße Pigmentgemisch kann sowohl vorteilhaft in der dekorativen als auch in der pflegenden Kosmetik eingesetzt werden. Die Einsatzkonzentration und das Mischungsverhältnis der roten Effektpigmente mit Komponente B, insbesondere organischen und anorganischen Farbpigmenten und Farbstoffen, natürlichen oder synthetischen Ursprungs, wie z.B. Chromoxid, Ultramarin, sphärischen SiO₂- oder TiO₂-Pigmenten, sind abhängig vom Anwendungsmedium und dem Effekt, der erzielt werden soll.

Das rote Effektpigment kann weiterhin mit handelsüblichen Füllstoffen gemischt werden. Als Füllstoffe sind z.B. zu nennen natürlicher und synthetischer Glimmer, Glasbeads oder Glaspulver, Nylon Powder, reine oder gefüllte Melaminharze, Talcum, Gläser, Kaolin, Oxide oder Hydroxide von Aluminium, Magnesium, Calcium, Zink, BiOCI, Bariumsulfat, Calciumsulfat, Calciumcarbonat, Magnesiumcarbonat, Kohlenstoff, sowie physikalische oder chemische Kombinationen dieser Stoffe.

Bezüglich der Partikelform des Füllstoffes gibt es keine Einschränkungen. Sie kann den Anforderungen gemäß z. B. plättchenförmig, sphärisch, nadelförmig, kristallin oder amorph sein.

Selbstverständlich können die roten Effektpigmente bzw. das erfindungsgemäße Pigmentgemisch in den Formulierungen auch mit jeder Art von kosmetischen Roh- und Hilfsstoffen kombiniert werden. Dazu gehören u.a. Öle, Fette, Wachse, Filmbildner, Tenside, Antioxidantien, wie z.B. Vitamin C oder Vitamin E, Stabilisatoren, Geruchsverstärker, Silikonöle, Emulgatoren, Lösemittel wie z.B. Ethanol, oder Ethylacetat oder Butylacetat, Konservierungsmittel und allgemein anwendungstechnische Eigenschaften bestimmende Hilfsstoffe, wie z.B. Verdicker und rheologische Zusatzstoffe wie etwa Bentonite, Hektorite, Siliciumdioxide, Ca-Silicate, Gelatinen, hochmolekulare Kohlenhydrate und/oder oberflächenaktive Hilfsmittel, etc.

Die die erfindungsgemäßen Pigmentgemische enthaltenden Formulierungen können dem lipophilen, hydrophilen oder hydrophoben Typ angehören. Bei heterogenen Formulierungen mit diskreten wässrigen und nichtwässrigen Phasen können die erfindungsgemäßen Pigmentgemische in jeweils nur einer der beiden Phasen enthalten oder auch über beide Phasen verteilt sein.

Die pH-Werte der Formulierungen können zwischen 1 und 14, bevorzugt zwischen 2 und 11 und besonders bevorzugt zwischen 5 und 8 liegen.

Den Konzentrationen der erfindungsgemäßen Pigmentgemische in der Formulierung sind keine Grenzen gesetzt. Sie können - je nach Anwendungsfall - zwischen 0,001 (rinse-off-Produkte, z.B. Duschgele) - 100 % (z.B. Glanzeffekt-Artikel für besondere Anwendungen) liegen.

Die erfindungsgemäßen Pigmente können weiterhin auch mit kosmetischen Wirkstoffen kombiniert werden. Geeignete Wirkstoffe sind z.B. Insect Repellents, anorganische UV-Filter, wie z.B. TiO₂, UV A/BC-Schutzfilter (z.B. OMC, B3, MBC), auch in verkapselter Form, Anti-Ageing-Wirkstoffe, Vitamine und deren Derivate (z.B. Vitamin A, C, E, etc.), Selbstbräuner (z.B. DHA, Erytrolyse u.a.) sowie weitere kosmetische Wirkstoffe, wie z.B. Bisabolol, LPO, VTA, Ectoin, Emblica, Allantoin, Bioflavanoide und deren Derivate.

Organische UV-Filter werden in der Regel in einer Menge von 0,5 bis 10 Gewichtsprozent, vorzugsweise 1 - 8 %, anorganische Filter von 0,1 bis 30% in kosmetische Formulierungen eingearbeitet.

Die erfindungsgemäßen Zubereitungen können darüber hinaus weitere übliche hautschonende oder hautpflegende Wirkstoffe enthalten. Dies können prinzipiell alle den Fachmann bekannten Wirkstoffe sein.

Besonders bevorzugte Wirkstoffe sind Pyrimidincarbonsäuren und/oder Aryloxime.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Up, Pflegecremes und Sonnenschutzpräparaten eingesetzt werden.

Als Anwendungsform der kosmetischen Formulierungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können beliebige übliche Trägerstoffe, Hilfsstoffe und gegebenenfalls weitere Wirkstoffe zugesetzt werden.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie z.B. Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Die kosmetischen Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-ÖI-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsionen werden besonders bevorzugt. Emulsionen, W/O-Emulsionen und O/W-Emulsionen sind in üblicher Weise erhältlich.

Weitere Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Feste Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen photochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Zubereitung vor oder nach dem Shamponieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Zubereitung mit Lichtschutzeigenschaften kann Adjuvantien enthalten, wie Grenzflächen aktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Ein weiterer Gegenstand der Erfindung sind somit auch Formulierungen enthaltend das erfindungsgemäße Pigmentgemisch nach Anspruch 1 in Kombination mit mindestens einem Bestandteil ausgewählt aus der Gruppe aus Absorptionsmitteln, Adstringenzien, antimikrobiellen Stoffen, Antioxidantien, Antiperspirantien, Antischaummitteln, Antistatika, Bindemitteln, biologischen Zusatzstoffen, Bleichmitteln, Chelatbildnern, Desodorierungsmitteln, Emollentien, Emulgatoren, Emulsionsstabilisatoren, Farbstoffen, Feuchthaltemitteln, Filmbildnern, Geruchsstoffen, Geschmackstoffen, Konservierungsstoffen, Korrosionsschutzmitteln, kosmetischen Ölen, Lösungsmitteln, Oxidationsmitteln, pflanzlichen Bestandteilen, Puffersubstanzen, Reduktionsmitteln, Schleifmitteln, Tensiden, Treibgasen, Trübungsmitteln, UV-Filtern und UV-Absorbern, Vergällungsmitteln, Viskositätsreglern und Vitaminen.

Die Einfärbung der Pharma- und Lebensmittelerzeugnisse erfolgt, indem das Pigmentgemisch bestehend vorzugsweise aus rotem Effektpigment und Färbemitteln, wie z.B. natürlichen oder naturidentischen Farbstoffen, in den gewünschten Mengenverhältnissen, dem einzufärbenden Erzeugnis in Mengen von 0,005 bis 15 Gew.%, vorzugsweise 0,01 bis 100 Gew.%, zugegeben wird.

Durch die Zumischung von für den Lebensmittelbereich zugelassenen, natürlichen bzw. naturidentischen Farbstoffen, organischen oder anorganischen Farbpigmenten oder färbenden natürlichen Frucht- und Pflanzenextrakten kann der rote Farbeffekt der Effektpigmente im Erzeugnis beeinflusst und gleichzeitig können neuartige changierende Farbeffekte erzielt werden.

Geeignete natürliche oder naturidentische Farbstoffe sind insbesondere E 101, E 104, E 110, E 124, E 131, E 132, E 140, E 141, E 151, E 160a. Weiterhin können auch andere Farbpigmente den plättchenförmigen Effektpigmenten beigemischt werden, wie z.B. E 171, E 172, E 153.

Der Anteil an Farbstoffen neben den roten Effektpigmenten bezogen auf das Lebensmittel- oder Pharmaprodukt liegt vorzugsweise im Bereich von 0,5 bis 25 Gew.%. Als Farbstoff können ebenfalls Frucht- und Pflanzenextrakte eingesetzt werden, wie z.B. Möhrensaft, Rote Beete-Saft, Holundersaft, Hibiskussaft, Paprikaextrakt, Aroniaextrakt.

Die Gesamtkonzentration aller Pigmente im zu pigmentierenden Erzeugnis sollte 50 Gew.% bezogen auf das Erzeugnis nicht übersteigen. Sie ist in der Regel abhängig vom konkreten Anwendungsfall.

Den Lebensmittel- und Pharmaprodukten können auch unterschiedliche Wirkstoffbeimischungen, wie z.B. Vitamine, Enzyme, Spurenelemente, Proteine, Kohlenhydrate, essentielle Fette und / oder Mineralien zugesetzt werden, wobei die Gesamtmenge an Wirkstoffen bezogen auf das Lebensmittel bzw. Pharmaprodukt 25 Gew.% nicht übersteigen sollte. Vorzugsweise beträgt die Menge an Wirkstoffen bzw. Wirkstoffgemischen 0,01 - 20 Gew.% bezogen auf das Produkt.

Die Einfärbung der Produkte erfolgt, indem das Effektpigmentgemisch allein oder in Kombination mit weiteren Pigmenten oder Färbemitteln direkt oder in Gegenwart von Wasser und/oder eines organischen Lösungsmittels in den gewünschten Mengenverhältnissen, gleichzeitig oder nacheinander, während oder nach ihrer Herstellung, vor oder nach der Formgebung (z.B. bei Extrusion, Pelletierung, Expondierung, Granulation, etc.) dem einzufärbenden Erzeugnis zugegeben wird. Eine Zumischung der roten Effektpigmente zu pulverförmigen bzw. losen Pulvern ist ebenfalls möglich.

Die roten Effektpigmente im Pigmentgemisch auch zur Färbung der Lebensmittel- und Pharmaprodukte nach der Formgebung auf die Oberfläche appliziert werden. Hierbei wird das rote Effektpigment in der Regel mit einem Auftragsmedium vermischt und anschließend mit geeigneten Auftrags- und Sprühvorrichtungen auf das Produkt aufgetragen. Das Auftrags- bzw. Überzugsmittel sorgt dann für die entsprechende Anhaftung der roten Pigmente auf der Produktoberfläche. Diese wird dann entsprechend gefärbt.

Bei der Einarbeitung in die Produktmatrix selbst, liegt die Einsatzmenge der roten Effektpigmente vorzugsweise bei 0,5 - 40 Gew.%, insbesondere bei 1 - 30 Gew.%. Bei der Oberflächenfärbung von Lebensmittel- und Pharmaprodukten liegt der Einsatzbereich in der verwendeten Farb- bzw. Überzugslösung bei 0,1 - 25 Gew.%, insbesondere bei 1 - 15 Gew. %. Bei der Verwendung der roten Effektpigmente in pulverförmigen Produkten liegt der Einsatzbereich bei 0,05 - 50 Gew. %, insbesondere bei 2 - 10 Gew.%.

Die Überzugslösungen enthalten vorzugsweise Wasser oder organische Lösemittel, wie z.B. Ethanol oder Isopropanol. Als Filmbildner wird in den Überzugslösungen vorzugsweise ein Cellulosederivat, wie z.B. Hydroxypropylmethylcellulose, eingesetzt. Insbesondere bevorzugt sind Auftragslösungen mit Cellulosederivaten, die statt Wasser 5 - 80 Gew. % eines geeigneten organischen Lösemittels enthalten.

Gegenüber wässrigen Überzugslösungen besitzen die alkoholischen, bzw. alkoholisch-wässrigen, Cellulose-haltigen Auftragslösungen deutliche anwendungstechnische Vorteile:
- Einsatz von kühlerer Trocknungsluft während des Sprühauftrags
- Färbung von wärmeempfindlichen Produkten, wie z.B. vitaminhaltigen Lebensmitteln, mit den roten Effektpigmenten ist sehr gut möglich.

Als zur Einfärbung geeignete Produkte sind insbesondere zu nennen Überzüge auf allen Arten von Lebensmitteln, insbesondere pigmentierte Zucker- und Schellacküberzüge (alkoholische und wässrige), Überzüge mit Ölen und Wachsen, mit Gummi Arabicum und mit Cellulosearten (z.B. HPMC = Hydroxypropylmethylcellulose), mit Stärke- und Eiweißderivaten, Carragenen und sonstigen dem Fachmann bekannten zum Überziehen geeigneten Substanzen. Hierbei wird das rote Effektpigment bzw. das Pigmentgemisch der Regel mit dem Auftragsmedium vermischt und anschließend mit geeigneten Auftrags- und Sprühvorrichtungen, oder per Hand auf dem Lebensmittel- oder Pharmaprodukt aufgetragen. Das Auftrags- bzw. Überzugsmittel sorgt dann für die entsprechende Anhaltung der Pigmente auf der Oberfläche des Lebensmittels oder Pharmaprodukts. Diese wird dann entsprechend gefärbt. Die Auftrags- und Überzugslösungen enthalten vorzugsweise 0,1 - 20 Gew.%, insbesondere 2-15 Gew.% an Effektpigmenten.

Bevorzugte Trockenpulvermischungen für Coatings enthalten ein Cellulosederivat, wie z.B. Hydroxypropylmethylcellulose, Natriumcarboxymethylcellulose, ein Trennmittel, wie z.B. Lecithin oder Stearinsäure, einen Glanzverstärker, wie z.B. Maltodextrin und/oder Dextrose, und das rote Effektpigment. Vorzugsweise enthalten derartige Trockenpulvermischungen das rote Effektpigment in Mengen von 0,01 - 50 Gew.%, insbesondere 0,5 - 40 Gew.% bezogen auf die Pulvermischung. Diesen Trockenpulvermischungen können je nach Bedarf noch Farbstoffe, Geschmackstoffe, Vitamine, Süßstoffe, etc., zugesetzt werden.

Für die Einfärbung bzw. für das Coaten geeignete Produkte sind beispielsweise Zuckerwaren, Kuchendekorationen, Komprimate, Dragees, Kaugummis, Gummiwaren, Fondanterzeugnisse, Marzipanerzeugnisse, Füllmassen, Kakao- und Fettglasuren, Schokolade und schokoladenhaltige Produkte, Speiseeis, Cerealien, Snackprodukte, Überzugsmassen, Tortenspiegel, Zuckerstreuseln, Nonpareilles, Gelee- und Gelatinewaren, Bonbons, Lakritze, Zuckerguss, Zuckerwatte, Fett-, Zucker- und Crememassen, Puddings, Desserts, Tortenguss, Kaltschalen, Limonaden und Brausegetränke, Getränke mit stabilisierenden Additiven, wie z. B. Carboxymethylcellulose, gesäuerte und ungesäuerte Milchprodukte, wie z. B. Quark, Joghurt, Käse, Käserinden, Wursthüllen, etc.

Bei dragierten bzw. gecoateten Lebensmittel- und Pharmaprodukten ist die Kombination der roten Effektpigmente mit Aromastoffen (Pulver- bzw. Flüssigaromen), Säuren und/oder mit Süßstoffen, wie z.B. Aspartam, möglich um den optischen Effekt auch geschmacklich zu betonen.

Gegenstand der Erfindung sind somit alle Formulierungen aus dem Lebensmittel- und Pharmabereich enthaltend das Pigmentgemisch allein oder in Kombination mit weiteren Pigmenten/Pigmentgemischen oder Farbstoffen (natürliche bzw. naturidentische) als Färbemittel.

Ein weiteres großes Einsatzgebiet liegt im Pharma- und OTC-Bereich zur Einfärbung bzw. als Überzug von Tabletten, Gelatinekapseln, Dragees, Salben, Hustensaft, etc. In Kombination mit üblichen Coatings wie Polymethacrylaten und Cellulosearten, z.B. HPMC, kann das rote Effektpigment vielfältig zur Einfärbung und Veredelung der Produkte eingesetzt werden.

Gegenstand der Erfindung sind somit auch Formulierungen enthaltend das erfindungsgemäße Pigmentgemisch.

Die nachfolgenden Beispiele sollen die Erfindung erläutern, ohne sie jedoch zu begrenzen.

### Beispiele

### Beispiel 1:

100 g SiO₂ Plättchen mit einer Dicke von 365 nm werden in 2 I vollentsalztem Wasser auf 75 °C erhitzt. Unter Rühren werden 1137 ml FeCl₃-Lösung (entspricht 132 % Fe₂O₃) zugegeben. Der pH-Wert der Reaktionsmischung wird durch Zugabe von Natronlauge (30 %ig) konstant bei 3 gehalten. Nach Zugabe der FeCl₃-Lösung wird der pH-Wert mit Natronlauge (30 %ig) auf pH 5 angehoben. Das Produkt wird abfiltriert und mit vollentsalztem Wasser nachgewaschen. Nach Trocknung bei 110 °C, wird bei 800 °C kalziniert.

Das Pigment besitzt folgende PDS (Particle Distribution Size)-Werte (gemessen mit Malvern Mastersizer 2000):
D₁₀ = 9,21
D₅₀ = 19,5
D₉₀ = 37,2

Das rote Effektpigment zeigt folgende L,a,b-Werte (gemessen mit Eta-Messgerät, Messwinkel 50°/140°):
L = 135,1
a = 102,6
b = 53,3

### Anwendungsbeispiele

### Beispiel A1: Eye shadow Gel

**Phase A**

| **Rohstoff** | **Bezugsquelle** | **INCI** | **[%]** |
|---|---|---|---|
| rotes Pigment gemäß Beispiel 1 | Merck KGaA/Rona® | CI 77491 (IRON OXIDES), SILICA | 15,00 |
| Micronasphere® M | Merck KGaA/Rona® | MICA, SILICA | 8,00 |
| Carbopol Ultrez 21 | Noveon | ACRYLATES/C10-30 ALKYL ACRYLATE CROSSPOLYMER | 0,40 |
| Citronensäure Monohydrat | Merck KGaA/Rona® | CITRIC ACID | 0,00 |
| Wasser | | AQUA (WATER) | ad 100 |

**Phase B**

| **Rohstoff** | **Bezugsquelle** | **INCI** | **[%]** |
|---|---|---|---|
| Glycerin | Merck KGaA/Rona® | GLYCERIN | 3,00 |
| Konservierungsmittel | | | q.s. |
| Triethanolamin | | TRIETHANOLAMINE | 0,70 |
| Wasser | | AQUA (WATER) | 13,00 |

**Phase C**

| **Rohstoff** | **Bezugsquelle** | **INCI** | **[%]** |
|---|---|---|---|
| Lubrajel DV | | PROPYLENE GLYCOL, POLYGLYCERYL-METHACRYLATE | 5,00 |

### Herstellung:

Rotes Pigment und Micronasphere® im Wasser der Phase A dispergieren. Mit einigen Tropfen Citronensäure ansäuern um die Viskosität zu vermindern, Carbopol unter Rühren einstreuen. Nach vollständiger Lösung die vorgelöste Phase B langsam einrühren und anschließend Phase C. Zum Schluss den pH-Wert zwischen 7,0 - 7,5 einstellen.

### Beispiel A2: Puder-Lidschatten

**Phase A**

| **Rohstoff** | **Bezugsquelle** | **INCI** | **[%]** |
|---|---|---|---|
| rotes Pigment gemäß Beispiel 1 | Merck KGaA/Rona® | CI 77491 (IRON OXIDES), SILICA | 30,00 |
| Talkum | Merck KGaA/Rona® | TALC | 49,50 |
| Magnesiumstearat | Merck KGaA/Rona® | MAGNESIUM STEARATE | 2,50 |
| Kartoffelstärke | Suedstaerke GmbH | SOLANUM TUBEROSUM (POTATO STARCH) | 7,50 |

**Phase B**

| **Rohstoff** | **Bezugsquelle** | **INCI** | **[%]** |
|---|---|---|---|
| Isopropylstearat | Cognis GmbH | ISOPROPYL STEARATE | 9,34 |
| Cetylpalmitat | Merck KGaA/Rona® | CETYL PALMITATE | 0,53 |
| Ewalin 1751 | H. Erhard Wagner GmbH | PETROLATUM | 0,53 |
| Propyl-4-hydroxybenzoat | Merck KGaA/Rona® | PROPYLPARABEN | 0,10 |

### Herstellung:

Bestandteile der Phase A zusammen geben und vormischen. Anschließend die Pudermischung unter Rühren tropfenweise mit der geschmolzenen Phase B versetzen. Die Puder werden bei 40-50 bar gepreßt.

### Suntest:

Um die Lichtstabilität zu beurteilen, wird eine Hälfte des Presspuders während der Belichtung abgedeckt. Die Puder werden dann für 8 Stunden in den Suntest (Hersteller: Hereaus Suntest CPS, Xenonstrahler mit 72 W/m²) gegeben.

Nach 8 Stunden Belichtung ist keine Verfärbung des Puders im Suntest zu beobachten. Das rote Pigment ist absolut lichtstabil.

### Beispiel A3: Lippenstift

**Phase A**

| **Rohstoff** | **Bezugsquelle** | **INCI** | **[%]** |
|---|---|---|---|
| rotes Pigment gemäß Beispiel 1 | Merck KGaA/Rona® | CI 77491 (IRON OXIDES), SILICA | 12,00 |
| Ronastar® Purple Sparks | Merck KGaA/Rona® | CALCIUM ALUMINUM BOROSILICATE, CI77891 (TITANIUM DIOXIDE), SILICA, TIN OXIDE | 3,00 |

**Phase B**

| **Rohstoff** | **Bezugsquelle** | **INCI** | **[%]** |
|---|---|---|---|
| Bienenwachs | Merck KGaA/Rona® | Cera Alba (Beeswax) | 8,75 |
| Paracera C44 | Paramelt | COPERNICIA CERIFERA (CARNAUBA WAX), CERESIN | 5,25 |
| Adeps Lanae | Henry Lamotte GmbH | LANOLIN | 3,50 |
| Isopropylmyristat | Cognis GmbH | Isopropyl Myristate | 5,60 |
| Paraffin dickflüssig | Merck KGaA/Rona® | PARAFFINUM LIQUIDUM (MINERAL OIL) | 2,10 |
| Rizinusöl | Henry Lamotte GmbH | RICINUS COMMUNIS (CASTOR OIL) | 59,65 |
| Oxynex K flüssig | Merck KGaA/Rona® | PEG-8, TOCOPHEROL, ASCORBYL PALMITATE ASCORBIC ACID, CITRIC ACID | 0,05 |
| Propyl-4-hydroxybenzoat | Merck KGaA/Rona® | PROPYLPARABEN | 0,10 |

### Herstellung:

Die Bestandteile der Phase B werden auf 75 °C erhitzt und aufgeschmolzen. Die Pigmente der Phase A werden zugegeben und alles gut durchrührt. Die Lippenstiftmasse wird dann in der auf 65 °C temperierten Gießapparatur 15 Minuten gerührt. Die homogene Schmelze wird in die auf 55 °C vorgewärmten Gießform gegossen. Anschließend kühlt man die Formen ab und entfernt die Gießlinge kalt. Nach Erwärmen der Lippenstifte auf Raumtemperatur werden die Lippenstifte kurz abgeflammt.

### Beispiel A4: Nagellack

| **Rohstoff** | **Bezugsquelle** | **INCI** | **[%]** |
|---|---|---|---|
| rotes Pigment gemäß Beispiel 1 | Merck KGaA/Rona® | CI 77491 (IRON OXIDES), SILICA | 2,00 |
| Nailsyn® Sterling 60 Silver | Merck KGaA/Rona® | CI 77163 (Bismuth OXYCHLORIDE), BUTYL ACETATE, NITROCELLULOSE, ISOPROPYL ALCOHOL, ETHYL ACETATE, STEARNALKONIUM HECTORITE | 1,00 |
| Thixotrope Nagellack - Base 155 | Durlin/Bergerac NC | BUTYL ACETATE, ETHYL ACETATE NITROCELLULOSE, ACETYL TRIBUTYL CITRATE, PHTHALIC ANHYDRIDE/TRIMEL LITIC ANHYDRIDE/GLYCOL S COPOLYMER, ISOPROPYL ALCOHOL, STEARALKONIUM HECTORITE, ADIPIC ACID/FUMARIC ACID/PHTHALIC ACID/TRICYCLODEC ANE DIMETHANOL COPOLYMER, CITRIC ACID | 97,00 |

### Herstellung:

Das Pigment und Nailsyn® Sterling 60 Silver werden zusammen mit der Lackbase eingewogen, gut mit einem Spatel von Hand vermischt und anschließend 10 min bei 1000 Upm gerührt.

### Beispiel A5: Volumenmascara

**Phase A**

| **Rohstoff** | **Bezugsquelle** | **INCI** | **[%]** |
|---|---|---|---|
| rotes Pigment gemäß Beispiel 1 | Merck KGaA/Rona® | CI 77491 (IRON OXIDES), SILICA | 10,00 |
| Satin Mica | Merck KGaA/Rona® | MICA | 2,00 |

**Phase B:**

| **Rohstoff** | **Bezugsquelle** | **INCI** | **[%]** |
|---|---|---|---|
| Dow Corning 556 | Dow Corning | PHENYL TRIMETHICONE | 2,00 |
| Tegosoft CT | Degussa-Goldschmidt AG | CAPRYLIC CAPRIC TRIGLYCERIDE | 2,50 |
| Syncrowachs HRC | Croda GmbH | TRIBEHENIN | 3,50 |
| Tegin M | Degussa-Goldschmidt AG | GLYCERYL STEARATE | 3,50 |
| Bienenwachs | Merck KGaA/Rona® | CERA ALBA (BEESWAX) | 3,00 |
| Stearinsäure | Merck KGaA/Rona® | STEARIC ACID | 5,00 |
| Phenonip | Nipa Laboratorien GmbH | PHENOXYETHANOL, BUTYLPARABEN, ETHYLPARABEN, PROPYLPARABEN, METHYLPARABEN | 0,80 |
| RonaCare® Tocopherolacetat | Merck KGaA/Rona® | TOCOPHEROLACETAT | 0,50 |
| Dermacryl 79 | Amerchol | ACRYLATES/OCTYL-ACRYLAMIDE COPOLYMER | 3,50 |

**Phase C**

| **Rohstoff** | **Bezugsquelle** | **INCI** | **[%]** |
|---|---|---|---|
| Wasser (demineralisiert) | | AQUA (WATER) | 59,15 |
| AMP Ultra PC 1000 | Angus Chemie GmbH | AMINOMETHYL PROPANOL | 1,25 |
| 1,3-Butandiol | Merck KGaA/Rona® | BUTYLENE GLYCOL | 1,00 |
| RonaCare® Biotin Plus | Merck KGaA/Rona® | UREA, DISODIUM PHOSPHATE, BIOTIN, CITRIC ACID | 0,50 |

**Phase D**

| **Rohstoff** | **Bezugsquelle** | **INCI** | **[%]** |
|---|---|---|---|
| Germall 115 | ISP Global Technologies | IMIDAZOLIDINYL UREA | 0,30 |
| Wasser (demineralisiert) | | AQUA (WATER) | 1,50 |

### Herstellung:

Alle Bestandteile der Phase B außer Demacryl 79 zusammen bei ca. 85 °C aufschmelzen, unter Rühren Demacryl 79 zugeben und 20 min rühren lassen bis alles homogen verteilt ist. Die Bestandteile der Phase C auf ca 85 °C erhitzen. Das rote Pigment der Phase A in Phase C einrühren. Phase C zu Phase B geben, weiter rühren und 1 min mit dem Ultra-Turrax T25 bei 8000 upm homogenisieren. Unter rühren abkühlen lassen und bei 40 °C Phase D zufügen.

### Beispiel A6: Seife

| **Rohstoff** | **Bezugsquelle** | **INCI** | **[%]** |
|---|---|---|---|
| rotes Pigment gemäß Beispiel 1 | Merck KGaA/Rona® | CI 77491 (IRON OXIDES), SILICA | 1,50 |
| Ronastar® Noble Sparks | Merck KGaA/Rona® | CALCIUM ALUMINUM BOROSILICATE, SILICA, CI 77891 (TITANIUM DIOXIDE), TIN OXIDE | 0,50 |
| Transparente Seifenbase | Jean Charles (USA) | SODIUM PALMATE, SODIUM LAURETH SULFATE, SODIUM STEARATE, SODIUM MYRISTATE, SODIUM COCOYL ISETHIONATE, TRIETHANOLAMINE, AQUA (WATER), GLYCERIN, SORBITOL, PROPYLENE GLYCOL, FRAGRANCE | 98,00 |

### Herstellung:

Alle Bestandteile werden homogen gemischt.

### Beispiel A7: Herstellung von Hartkaramellen

| **Rohstoff** | **%** | **Bezugsquellen:** |
|---|---|---|
| Zucker | 41 % | Fa. Südzucker |
| Wasser | 17,118 % | |
| Glucosesirup | 41 % C* Sweet | Fa. Cerestar, Krefeld |
| rotes Pigment gemäß Beispiel 1 | 0,082 % (0,1 % bezogen auf die Gießmasse) | Fa. Merck KGaA, Darmstadt |
| E 104 1 : 100 verd. | 0,4 % Sikovit | Fa. BASF, Ludwigshafen |
| Aroma | 0,4 % (Banane 9/030388) | Fa. Dragaco, Holzminden |

Der Zucker wird mit dem Wasser auf 100 °C erhitzt und danach mit Glucosesirup versetzt. Die Lösung wird anschließend auf 145 °C erhitzt. Nach Zugabe des roten Effektpigments, der Farblösung und dem Aroma wird die Karamellösung mit einem Gießtrichter in gefettete Formen gegossen. Zuletzt läßt man zwei Stunden abkühlen. Das rote Pigment kann sowohl mit dem Zucker vermischt werden als auch mit dem Glukosesirup vermischt zugegeben werden. Diese Variante enthält keine Säure, da hierdurch die Karamelisation zu stark würde.

### Beispiel A8: Herstellung von Gelatineartikeln

| **Rohstoff** | **%** | **Bezugsquellen:** |
|---|---|---|
| Wasser | 10,48 % | |
| Zucker | 31,45 % | Fa. Südzucker |
| Glukosesirup | 31,45 % C*Sweet | Fa. Cerestar, Krefeld |
| rotes Pigment gemäß Beispiel 1 | 0,38 % (0,4% bezogen auf die Gießmasse) | Fa. Merck KGaA, Darmstadt |
| Zitronensäure 1:1 verd. | 2,51 % | Fa. Merck KGaA, Darmstadt |
| Gelatine | 7,86 % 260 Bloom | Fa. DGF, Eberbach |
| Wasser | 15,748 % | |
| Aroma | 0,122 % (schwarze Johannisbeere 9/695750) | Fa. Dragoco, Holzminden |

Zunächst wird die Gelatine mit der doppelten Menge an Wasser bei 60 °C eingeweicht. Zucker und Wasser werden auf 100 °C erhitzt, dann wird der Glucosesirup zugegeben. Man erhitzt weiter auf 120 °C und läßt dann auf ca. 85 °C abkühlen. Das rote Effektpigment, die Zitronensäure, das Aroma und die Gelatinelösung werden untergerührt, und das entlüftete Gelatinegemisch wird mit dem Gießtrichter in gefettete Formen abgefüllt. Das Produkt läßt man ca. 16 Stunden abkühlen.

Weitere Ausführungsformen:
- Das rote Effektpigment kann hierbei wieder direkt schon mit dem Zucker vermischt werden oder mit dem Glukosesirup eingebracht werden.
- Anstelle des Gießens in Formen kann auch die traditionelle Technik mit Negativformen in Formpuder zur Herstellung von Gelatineartikeln hierbei verwendet werden.

### Beispiel A9: Coating von Tabletten

### a) Einwaage 1 kg weiße Tabletten d=8mm, G=200mg

| Lösung für das Filmcoating: | | |
|---|---|---|
| 6 % | Sepifilm Lp10 (Gemisch aus Hydroxypropylmethylcellulose, Stearinsäure und mikrokristalliner Cellulose | Fa. Seppic |
| 5 % | rotes Pigment gemäß Beispiel 1 | Fa. Merck KGaA, Darmstadt |
| 89 % | Wasser | |

Gesamtauftragsmenge: 200 g
Dies entspricht 1,2 mg Polymer/cm² Tablettenoberfläche

Herstellung der Film-Coating-Lösung:
- Das rote Effektpigment wird in Wasser eingerührt. Anschließend fügt man gegebenenfalls zusätzliche Farbstoffe hinzu. Schließlich streut man den Filmbildner (HPMC) in die Suspension ein. Durch die ansteigende Viskosität bedingt, muß auch die Rührgeschwindigkeit dementsprechend erhöht werden. Nach ca. 40-60 Minuten ist die HPMC vollständig gelöst und die Lösung kann nun auf die Tabletten aufgesprüht werden.
- Der Sprühauftrag erfolgt mittels gängigem Standard-Coating-Verfahren.

## Patentansprüche

1. Pigmentgemisch bestehend aus mindestens zwei Komponenten A und B, **dadurch gekennzeichnet, dass** Komponente A Effektpigmente sind basierend auf transparenten undotierten SiO₂-Plättchen, die mit einer Eisenoxidschicht umhüllt sind, alle SiO₂-Plättchen eine identische Dicke aufweisen und folgenden Aufbau besitzen:
SiO₂-Plättchen der Dicke 250-400 nm,
Fe₂O₃-Schicht der Dicke 60-250 nm,
wobei die Dicken der SiO₂-Plättchen und der Eisenoxidschicht derart gewählt werden, dass die Gesamtdicke der roten Effektpigmente nicht größer als 500 (± 30) nm ist,
und Komponente B anorganische Pigmente, organische Pigmente, Farbstoffe und/oder Füllstoffe sind, die aus plättchenförmigen, nadelförmigen, sphärischen oder unregelmäßig geformten Partikeln bestehen und die Komponente A und Komponente B im Massenverhältnis 99 : 1 bis 50 : 50 gemischt sind.

2. Pigmentgemisch nach Anspruch 1, **dadurch gekennzeichnet, dass** bei den Effektpigmenten der Komponente A die SiO₂-Plättchen eine Dicke von 300-370 nm besitzen.

3. Pigmentgemisch nach Anspruch 1 oder 2, **dadurch gekennzeichnet, dass** das Farbmittel der Komponente B ein Perlglanzpigment, ein Mehrschichtpigment und/oder ein Interferenzpigment oder deren Gemisch ist.

4. Verwendung des Pigmentgemisches nach einem oder mehreren der Ansprüche 1 bis 3 in kosmetischen Formulierungen und im Lebensmittel- und Pharmabereich.

5. Verwendung des Pigmentgemisches nach einem oder mehreren der Ansprüche 1 bis 3 in Lebensmittel- und Pharmaprodukten, **dadurch gekennzeichnet, dass** das Pigmentgemisch allein oder in Kombination mit weiteren Pigmenten und/oder Farbmitteln in Kombination mit Aromastoffen und/oder Süßstoffen eingesetzt wird.

6. Formulierungen enthaltend ein Pigmentgemisch nach einem oder mehreren der Ansprüche 1 bis 3.

7. Formulierungen nach Anspruch 6 enthaltend das Pigmentgemisch und mindestens einen Bestandteil ausgewählt aus der Gruppe der Absorptionsmittel, Adstringenzien, antimikrobiellen Stoffen, Antioxidantien, Antiperspirantien, Antischaummitteln, Antistatika, Bindemitteln, biologischen Zusatzstoffen, Bleichmitteln, Chelatbildnern, Desodorierungsmitteln, Emollentien, Emulgatoren, Emulsionsstabilisatoren, Farbstoffen, Feuchthaltemitteln, Filmbildnern, Geruchsstoffen, Geschmackstoffen, Konservierungsstoffen, Korrosionsschutzmitteln, kosmetischen Ölen, Lösungsmitteln, Oxidationsmitteln, pflanzlichen Bestandteilen, Puffersubstanzen, Reduktionsmitteln, Schleifmitteln, Tensiden, Treibgasen, Trübungsmitteln, UV-Filtern und UV-Absorbern, Vergällungsmitteln, Viskositätsreglern und Vitaminen.

8. Verfahren zur Herstellung der Formulierungen nach Anspruch 6 oder 7, **dadurch gekennzeichnet, dass** die Komponenten A und B des Pigmentgemisches nach einem oder mehreren der Ansprüche 1 bis 3 gleichzeitig, nacheinander oder als Gemisch dem Anwendungssystem zugegeben werden.

## Claims

1. Pigment mixture consisting of at least two components A and B, **characterised in that** component A comprises effect pigments based on transparent, undoped SiO₂ flakes which are covered with an iron-oxide layer, all SiO₂ flakes have an identical thickness and the following structure:
SiO₂ flake having a thickness of 250-400 nm,
Fe₂O₃ layer having a thickness of 60-250 nm,
where the thicknesses of the SiO₂ flakes and of the iron-oxide layer are selected in such a way that the total thickness of the red effect pigments is not greater than 500 (± 30) nm,
and component B comprises inorganic pigments, organic pigments, dyes and/or fillers which consist of flake-form, needle-shaped, spherical or irregularly shaped particles and component A and component B are mixed in the weight ratio 99 : 1 to 50 : 50.

2. Pigment mixture according to Claim 1, **characterised in that** in the effect pigments of component A, the SiO₂ flakes have a thickness of 300-370 nm.

3. Pigment mixture according to Claim 1 or 2, **characterised in that** the colorant of component B is a pearlescent pigment, a multilayered pigment and/or an interference pigment or a mixture thereof.

4. Use of the pigment mixture according to one or more of Claims 1 to 3 in cosmetic formulations and in the foods and pharmaceuticals sector.

5. Use of the pigment mixture according to one or more of Claims 1 to 3 in food and pharmaceutical products, **characterised in that** the pigment mixture is employed alone or in combination with further pigments and/or colorants in combination with aroma substances and/or sweeteners.

6. Formulations comprising a pigment mixture according to one or more of Claims 1 to 3.

7. Formulations according to Claim 6 comprising the pigment mixture and at least one constituent selected from the group of the absorbents, astringents, antimicrobial substances, antioxidants, antiperspirants, antifoaming agents, antistatics, binders, biological additives, bleaching agents, chelating agents, deodorants, emollients, emulsifiers, emulsion stabilisers, dyes, humectants, film formers, odour substances, flavour substances, preservatives, corrosion-protection agents, cosmetic oils, solvents, oxidants, vegetable constituents, buffer substances, reducing agents, abrasives, surfactants, propellant gases, opacifiers, UV filters and UV absorbers, denaturing agents, viscosity regulators and vitamins.

8. Process for the preparation of the formulations according to Claim 6 or 7, **characterised in that** components A and B of the pigment mixture according to one or more of Claims 1 to 3 are added to the application system simultaneously, successively or as a mixture.

## Revendications

1. Mélange de pigments constitué par au moins deux composants A et B, **caractérisé en ce que** le composant A comprend des pigments d'effet qui sont basés sur des flocons de SiO₂ non dopés transparents qui sont recouverts d'une couche d'oxyde de fer, tous les flocons de SiO₂ présentent une épaisseur identique et la structure qui suit :
flocon de SiO₂ présentant une épaisseur de 250 - 400 nm ;
couche de Fe₂O₃ présentant une épaisseur de 60 - 250 nm ;
où les épaisseurs des flocons de SiO₂ et de la couche d'oxyde de fer sont sélectionnées de telle sorte que l'épaisseur totale des pigments d'effet rouge ne soit pas supérieure à 500 (± 30) nm,
et le composant B comprend des pigments inorganiques, des pigments organiques, des colorants et/ou des agents de remplissage qui sont constitués par des particules sous forme de flocons, en forme d'aiguilles, sphériques ou de forme irrégulière, et le composant A et le composant B sont mélangés selon le rapport en poids de 99 : 1 à 50 : 50.

2. Mélange de pigments selon la revendication 1, **caractérisé en ce que**, dans les pigments d'effet du composant A, les flocons de SiO₂ présentent une épaisseur de 300 - 370 nm.

3. Mélange de pigments selon la revendication 1 ou 2, **caractérisé en ce que** le colorant du composant B est un pigment perlescent, un pigment multicouche et/ou un pigment d'interférence ou un mélange de ceux-ci.

4. Utilisation du mélange de pigments selon une ou plusieurs des revendications 1 à 3 dans les formulations cosmétiques et dans le secteur des produits alimentaires et des produits pharmaceutiques.

5. Utilisation du mélange de pigments selon une ou plusieurs des revendications 1 à 3 dans les produits alimentaires et pharmaceutiques, **caractérisé en ce que** le mélange de pigments est utilisé seul ou en combinaison avec d'autres pigments et/ou colorants en combinaison avec des substances aromatiques et/ou des édulcorants.

6. Formulations comprenant un mélange de pigments selon une ou plusieurs des revendications 1 à 3.

7. Formulations selon la revendication 6, comprenant le mélange de pigments et au moins un constituant qui est sélectionné parmi le groupe qui est constitué par les absorbants, les astringents, les substances antimicrobiennes, les antioxydants, les produits anti-sudorifiques, les agents anti-moussants, les agents antistatiques, les agents de liaison, les additifs biologiques, les agents de blanchiment, les agents chélatants, les déodorants, les agents adoucissants, les émulsifiants, les stabilisateurs d'émulsion, les colorants, les agents humidifiants, les agents filmogènes, les substances odoriférantes, les substances aromatisantes, les conservateurs, les agents de protection contre la corrosion, les huiles cosmétiques, les solvants, les oxydants, les constituants végétaux, les substances tampon, les agents de réduction, les abrasifs, les agents tensioactifs, les gaz de propulsion, les opacifiants, les filtres UV et les absorbeurs d'UV, les agents de dénaturation, les régulateurs de viscosité et les vitamines.

8. Procédé pour la préparation des formulations selon la revendication 6 ou 7, **caractérisé en ce que** les composants A et B du mélange de pigments selon une ou plusieurs des revendications 1 à 3 sont ajoutés au système d'application simultanément, successivement ou en tant que mélange.
